(19) Europäisches Patentamt
European Patent Office
Office européen des brevets

(11) Veröffentlichungsnummer: **0 039 807**
**B1**

(12) **EUROPÄISCHE PATENTSCHRIFT**

(45) Veröffentlichungstag der Patentschrift: **15.02.84**

(51) Int. Cl.³: **A 61 K 7/13**

(21) Anmeldenummer: **81103092.3**

(22) Anmeldetag: **24.04.81**

(54) **Haarfärbemittel.**

(30) Priorität: **02.05.80 DE 3016905**

(43) Veröffentlichungstag der Anmeldung: **18.11.81 Patentblatt 81/46**

(45) Bekanntmachung des Hinweises auf die Patenterteilung: **15.02.84 Patentblatt 84/7**

(84) Benannte Vertragsstaaten: **AT BE CH DE FR GB IT LI NL SE**

(56) Entgegenhaltungen:
**FR - A - 1 506 234**
**FR - A - 2 302 725**
**FR - A - 2 302 726**
**FR - A - 2 352 542**
**GB - A - 749 045**

**CHEMICAL ABSTRACTS, Band 83, Nr. 1, 7. Juli 1975, Seite 777, Nr. 9372u, Columbus, Ohio, U.S.A. N. SCHAMP et al.: "Two-step synthesis of 2-chlororesorcinols from 1,3-cyclohexanediones"**
**BULLETIN SOC. CHIM. BELG., Band 73, 1964, Seiten 81-95,Oxford, G.B. N. SCHAMP et al.: "Halogen substituted 1,3-cyclohexanediones"**
**JOURNAL OF THE INDIAN CHEMICAL SOCIETY, Band 14, 1937, Seiten 725-732, Calcutta, IN. D. CHAKRAVARTI et al.: "Synthesis of coumarins from phenols and acetoacetic esters. Constitution of halogenated resorcins and orcins"**
**TETRAHEDRON, Band 29, 1973, Seiten 3857-3859, Pergamon Press, Oxford, G.B. N. SCHAMP et al.: "New synthesis of 2-substituted recorcinols"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Rose, David, Dr., Holbeinweg 7, D-4010 Hilden (DE)**
Erfinder: **Maak, Norbert, Dr., Liebigstrasse 18, D-4040 Neuss (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Haarfärbemittel

Gegenstand der Erfindung sind Mittel zur oxidativen Färbung von Haaren auf Basis von Chlorresorcinen als Farbstoffkomponenten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, dass auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Farbnuance oder gar Farbumschläge in andere Farbtöne zu vermeiden.

Es waren zwar bereits Chlorresorcine als Haarfarbstoff-Vorprodukte bekannt, z.B. aus GB-PS 749 045 das 5-Chlorresorcin und aus FR-A 2 352 542 das 4-Chlorresorcin. Diese Resorcinderivate erfüllen jedoch noch nicht die an einen Kuppler gestellten Anforderungen in bezug auf Farbintensität und Echtheit der damit erzielbaren Färbungen.

2-Chlorresorcin war als Kuppler für Diazoniumsalze aus FR-A 1 506 243 bekannt. Die Verwendung von Chlor-m-aminophenolen als Kuppler für Oxidationshaarfärbungen war aus FR-A 2 302 725 und FR-A 2 302 726 bekannt. Aus den genannten Druckschriften war jedoch kein Hinweis auf den Gegenstand der vorliegenden Erfindung zu entnehmen.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, dass Haarfärbemittel auf Basis von Oxidationsfarbstoffen mit einem Gehalt an Chlorresorcinen der Formel

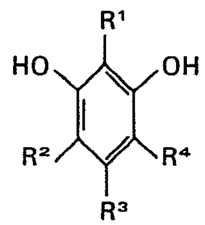

in der die Substituenten $R^1$ bis $R^4$ Chloratome, Wasserstoffatome oder Methylgruppen darstellen, wobei die Kombinationen

a) $R^1=Cl$, $R^2=H$, $R^3=H$ und $R^4=H$,
b) $R^1=Cl$, $R^2=H$, $R^3=CH_3$ und $R^4=H$,
c) $R^1=Cl$, $R^2=H$, $R^3=CH_3$ und $R^4=CH_3$,
d) $R^1=H$, $R^2=H$, $R^3=CH_3$ und $R^4=Cl$,
e) $R^1=CH_3$, $R^2=Cl$, $R^3=H$ und $R^4=Cl$,

gelten als Kupplerkomponenten und den in Oxidationshaarfarben üblichen Entwicklersubstanzen den gestellten Anforderungen in besonders hohem Masse gerecht werden.

Bei ihrem Einsatz als Kupplersubstanzen liefern die in den erfindungsgemässen Haarfärbemitteln enthaltenen Chlorresorcine mit den im allgemeinen für die Oxidationshaarfärbung verwendeten Entwicklerkomponenten sehr intensive Rot- bis Dunkelbrauntöne mit sehr guten Echtheitseigenschaften. Darüber hinaus zeichnen sich die erfindungsgemäss einzusetzenden Verbindungen durch eine sehr gute Löslichkeit in Wasser, eine gute Lagerstabilität und toxikologische sowie dermatologische Unbedenklichkeit aus und stellen somit eine wesentliche Bereicherung der oxidativen Haarfärbemöglichkeiten dar.

Die in den erfindungsgemässen Haarfärbemitteln als Kupplersubstanzen enthaltenen Chlorresorcine stellen literaturbekannte Verbindungen dar. Ihre Herstellung ist beispielsweise beschrieben in Bull. Soc. Chim. Belg. *73* (1964), 34, 81, Meded. Fac. Landbouwwet., Rijksuniv. Gent *39*, (1974), 1645, J. Ind. Chem. Soc. *14* (1937), 730 und Tetr. *29*, 3859, ohne dass dort ihre Einsatzmöglichkeiten als Haarfarbstoffkomponenten angesprochen werden.

Bei den Kupplersubstanzen, die in den erfindungsgemässen Haarfärbemitteln enthalten sind, handelt es sich um die Verbindungen 2-Chlorresorcin, 2-Chlor-5-methyl-resorcin, 2-Chlor-4,5-dimethylresorcin, 4-Chlor-5-methylresorcin und 4,6-Dichlor-2-methylresorcin.

Als Beispiele für Entwicklerkomponenten, die in den erfindungsgemässen Haarfärbemitteln eingesetzt werden können, sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlor-p-phenylendiamin, N,N-Bis-hydroxyethylamino-p-phenylendiamin, Methoxy-p-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoff-atomen darstellt, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 2-Methylamino-4,5,6-triaminopyrimidin, 4,5-Diamino-2,6-bismethyl-aminopyrimidin, 2,5-Diamino-4-diethylamino-6-methyl-aminopyrimidin, 2,4,5-Triamino-6-dimethylaminopyrimidin, 2,4,5-Triamino-6-piperidino-pyrimidin, 2,4,5-Triamino-6-anilino-pyrimidin, 2,4,5-Triamino-6-morpholino-pyrimidin, 2,4,5-Triamino-6-β-hydroxyethylaminopyrimidin anzuführen.

In den erfindungsgemässen Haarfärbemitteln werden die Entwicklerkomponenten im allgemeinen in etwa molaren Menge, bezogen auf die verwendeten Kupplersubstanzen, eingesetzt. Wenn sich auch der molare Einsatz als zweckmässig erweist, so ist es jedoch nicht nachteilig, wenn die Kupplerkomponente in einem gewissen Überschuss oder Unterschuss zum Einsatz gelangt.

Es ist ferner nicht erforderlich, dass die Entwicklerkomponente und die Kupplersubstanz einheitliche Produkte darstellen, vielmehr können sowohl die Entwicklerkomponente als auch die Kupplersubstanz Gemische der vorstehend genannten entsprechenden Verbindungen darstellen.

Darüber hinaus können die erfindungsgemässen Haarfärbemittel andere bekannte und übliche Kupplersubstanzen sowie auch gegebenenfalls übliche direktziehende Farbstoffe im Gemisch enthalten, falls dies zur Erzielung gewisser Farbnuancen erforderlich ist.

Die oxidative Kupplung, das heisst die Entwicklung der Färbung, kann grundsätzlich wie bei anderen Oxidations-haarfarbstoffen auch, durch Luftsauerstoff erfolgen. Zweckmässigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie gemische aus derartigen Wasserstoffperoxidanlagerungsverbindungen mit Kaliumperoxiddisulfat in Betracht.

Die erfindungsgemässen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemässen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40° C. Nach einer Einwirkungsdauer von circa 30 Minuten wird das Haarfärbemittel vom zu färbenden Haar durch Spülen entfernt. Hernach wird das Haar mit einem milden Shampoo nachgewaschen und getrocknet. Mit den erfindungsgemässen Haarfärbemitteln lässt sich unter Einsatz unterschiedlicher Entwicklerkomponenten und Kupplersubstanzen ein breites Spektrum an Rot- bis Dunkelbrauntönen abdecken. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

*Beispiele:*

Die in den nachfolgenden Beispielen als Kupplerkomponenten eingesetzten Chlorresorcine wurden, soweit es sich nicht um Handelsprodukte handelt, entsprechend den in den vorstehend genannten Literaturstellen enthaltenen Angaben hergestellt.

Als Kupplerkomponenten dienten folgende Verbindungen:

K1: 2-Chlorresorcin
K2: 2-Chlor-5-methylresorcin
K3: 2-Chlor-4,5-dimethylresorcin
K4: 4-Chlor-5-methylresorcin
K5: 4,6-Dichlor-2-methylresorcin

Als Entwicklerkomponenten wurden in Kombination mit den Chlorresorcinen in den folgenden Beispielen die nachstehend genannten Verbindungen eingesetzt.

E1: p-Toluylendiamin
E2: 2,4,5,6-Tetraaminopyrimidin
E3: p-Phenylendiamin
E4: N,N-Dimethyl-p-phenylendiamin
E5: 2-Chlor-p-phenylendiamin
E6: 2,5-Diaminoanisol
E7: p-Aminophenol
E8: N-Methyl-p-phenylendiamin

E9: N-Ethyl-N-β-hydroxyethyl-p-phenylendiamin

E10: N,N-Bis-(β-hydroxyethyl)-p-phenylendiamin

E11: 2-Methylamino-4,5,6-triaminopyrimidin

Die erfindungsgemässen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$,

10 Gewichtsteilen Fettalkoholsulfat (Natriumsalz) Kettenlänge $C_{12}$-$C_{18}$,

75 Gewichtsteilen Wasser,

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1%iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit zusätzlichem Oxidationsmittel wurde auf zu 90% ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschliessend getrocknet. Die dabei erhaltenen Färbungen sind nachstehender Tabelle 1 zu entnehmen.

*Tabelle 1*

| Beispiel | a) Kuppler | b) Entwickler | mit 1%iger $H_2O_2$-Lösung erhaltener Farbton |
|---|---|---|---|
| 1 | K1 | E1 | graubraun |
| 2 | K1 | E2 | hellbraun |
| 3 | K2 | E1 | haarbraun |
| 4 | K2 | E2 | graurot |
| 5 | K3 | E1 | teakholzfarbig |
| 6 | K3 | E2 | rotbraun |
| 7 | K4 | E1 | graubraun |
| 8 | K4 | E2 | rotbraun |
| 9 | K4 | E3 | braungrau |
| 10 | K4 | E4 | graubraun |
| 11 | K5 | E1 | dunkelbraun |
| 12 | K5 | E1 | rotviolett |
| 13 | K5 | E5 | teakholzfarbig |
| 14 | K5 | E6 | dunkelmagenta |
| 15 | K5 | E7 | gelbbraun |
| 16 | K5 | E8 | nutria |
| 17 | K5 | E9 | braungrau |
| 18 | K5 | E10 | braungrau |
| 19 | K5 | E11 | braunrot |

## Patentansprüche

1. Haarfärbemittel auf Basis von Oxidationsfarbstoffen, gekennzeichnet durch einen Gehalt an Chlorresorcinen der Formel

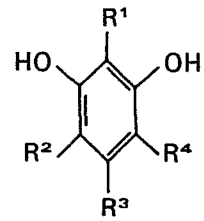

in der die Substituenten $R^1$ bis $R^4$ Chloratome, Wasserstoffatome oder Methylgruppen darstellen, wobei die Kombinationen

a) $R^1$=Cl, $R^2$=H, $R^3$=H und $R^4$=H,
b) $R^1$=Cl, $R^2$=H, $R^3$=$CH_3$ und $R^4$=H,
c) $R^1$=Cl, $R^2$=H, $R^3$=$CH_3$ und $R^4$=$CH_3$,
d) $R^1$=H, $R^2$=H, $R^3$=$CH_3$ und $R^4$=Cl,
e) $R^1$=$CH_3$, $R^2$=Cl, $R^3$=H und $R^4$=Cl

gelten, als Kupplerkomponenten und den in Oxidationshaarfarben üblichen Entwicklersubstanzen.

2. Haarfärbemittel nach Anspruch 1, gekennzeichnet durch einen Gehalt an Entwickler-Kuppler-Kombination von 0,2 bis 5 Gewichtsprozent, vorzugsweise 1 bis 3 Gewichtsprozent.

## Claims

1. Hair dyes based on oxidation dyes, characterized by a content of chlororesorcinols corresponding to the following formula

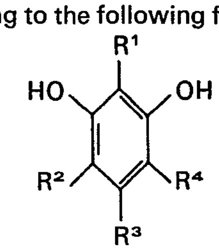

in which the substituents $R^1$ to $R^4$ represent chlorine atoms, hydrogen atoms or methyl groups, the following combinations

a) $R^1$=Cl, $R^2$=H, $R^3$=H and $R^4$=H,
b) $R^1$=Cl, $R^2$=H, $R^3$=$CH_3$ and $R^4$=H,
c) $R^1$=Cl, $R^2$=H, $R^3$=$CH_3$ and $R^4$=$CH_3$,
d) $R^1$=H, $R^2$=H, $R^3$=$CH_3$ and $R^4$=Cl,
e) $R^1$=$CH_3$, $R^2$=Cl, $R^3$=H and $R^4$=Cl.

applying, as coupler components and the developer substances normally used in oxidation hair dyes.

2. Hair dyes as claimed in Claim 1, characterized by a content of developer-coupler combination of from 0.2 to 5% by weight and preferably from 1 to 3% by weight.

## Revendications

1. Teinture pour cheveux à base de colorants par oxydation, caractérisée en ce qu'elle contient des chlororésorcinols de formule

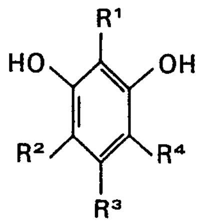

dans laquelle les symboles $R^1$ à $R^4$ représentent des atomes de chlore, des atomes d'hydrogène ou des groupes méthyle, les combinaisons suivantes étant valables:

a) $R^1=Cl$, $R^2=H$, $R^3=H$ et $R^4=H$,
b) $R^1=Cl$, $R^2=H$, $R^3=CH_3$ et $R^4=H$,
c) $R^1=Cl$, $R^2=H$, $R^3=CH_3$ et $R^4=CH_3$,
d) $R^1=H$, $R^2=H$, $R^3=CH_3$ et $R^4=Cl$,
e) $R^1=CH_3$, $R^2=Cl$, $R^3=H$ et $R^4=Cl$

en tant que copulants, et les substances révélatrices usuelles dans les teintures pour cheveux par oxydation.

2. Teinture pour cheveux selon la revendication 1, caractérisée en ce qu'elle contient la combinaison révélateur-copulant en quantité de 0,2 à 5% en poids, de préférence de 1 à 3% en poids.